(19) Europäisches Patentamt

European Patent Office

Office européen des brevets

(11) Veröffentlichungsnummer: **0 367 010 B1**

(12) **EUROPÄISCHE PATENTSCHRIFT**

(45) Veröffentlichungstag der Patentschrift: **07.12.94**

(51) Int. Cl.⁵: **C07D 209/50**, C07C 211/35

(21) Anmeldenummer: **89119201.5**

(22) Anmeldetag: **17.10.89**

Die Akte enthält technische Angaben, die nach dem Eingang der Anmeldung eingereicht wurden und die nicht in dieser Patentschrift enthalten sind.

(54) **Verfahren zur Herstellung von Cyclopropylamin.**

(30) Priorität: **29.10.88 DE 3836917**

(43) Veröffentlichungstag der Anmeldung:
**09.05.90 Patentblatt 90/19**

(45) Bekanntmachung des Hinweises auf die
Patenterteilung:
**07.12.94 Patentblatt 94/49**

(84) Benannte Vertragsstaaten:
**BE CH DE FR GB IT LI**

(56) Entgegenhaltungen:
DE-A- 1 939 759
FR-A- 2 093 472

**ROGER ADAMS "ORGANIC REACTIONS",
Band III, Seiten 267-270, 280-283, 288, 1946,
John Wiley & Sons, New York, U.S.A. E.S.
Wallis et al.**

**JUSTUS LIEBIGS ANNALEN DER CHEMIE,
Band 499, Seiten 1-25, 1932, Verlag Chemie,
Weinheim/Bergstr. und Berlin, DE; P. LIPP et
al.**

**Beyer, "Lehrbuch der organischen Chemie",
18. Auflage, 1976, Seite 147**

(73) Patentinhaber: **BAYER AG**

**D-51368 Leverkusen (DE)**

(72) Erfinder: **Diehl, Herbert, Dr.
Walter-Flex-Strasse 17
D-5090 Leverkusen 1 (DE)**
Erfinder: **Blank, Heinz Ulrich, Dr.
Am Geusfelde 35
D-5068 Odenthal-Glöbusch (DE)**
Erfinder: **Ritzer, Edwin, Dr.
Ludwig-Bette-Weg 4
D-4390 Gladbeck (DE)**

**Beschreibung**

Die vorliegende Erfindung bezieht sich auf ein Verfahren zur Herstellung von Cyclopropylamin aus Cyclopropancarbonsäureamid durch den sogenannten Hofmann-Abbau.

Cyclopropylamin ist ein wichtiges Zwischenprodukt bei der Herstellung biologisch aktiver Substanzen.

Der sogenannte Hofmann-Abbau der Umlagerung von Säureamiden unter Decarboxylierung zu primären Aminen ist grundsätzlich bekannt. Dieses Verfahren ist auch schon auf die Herstellung von Cyclopropylamin angewandt worden (DE-OS 19 39 759). Hierbei wird eine Suspension des Cyclopropancarbonsäureamids in Wasser mit Natriumhypochloritlösung bei 0°C versetzt; nach dem Zusammengeben der genannten Stoffe wird eine Nachreaktionszeit von 45 Minuten ebenfalls bei 0°C angeschlossen. Daraufhin wird 20 %ige Natronlauge unter Kühlung zugesetzt und nach einer weiteren Nachreaktionszeit von 30 Minuten wird die Reaktionslösung 2 Stunden lang auf 45-50°C erwärmt. Bis zu diesem Punkt, an dem erst eine lagerstabile wäßrige Lösung das Cyclopropylamins erhalten wird, vergehen 4 Stunden. Durch destillative Aufarbeitung wird eine 25-30 %ige wäßrige Lösung von Cyclopropylamin erhalten. Der Umwandlungsgrad wird mit 85-95 % angegeben.

Die relativ lange Gesamtreaktionszeit einerseits und die trotz des Arbeitens bei 0°C andererseits ablaufenden Nebenreaktionen, die sich in Minderausbeuten äußern, zeigen die Schwierigkeiten dieses Verfahrens.

Es wurde nun gefunden, daß es erhebliche Vorteile mit sich bringt, wenn man das Cyclopropancarbonsäureamid in Form einer wäßrigen Lösung anstelle einer Suspension einsetzt, obwohl hierbei eine geringere Raumausbeute in Kauf genommen werden muß. Eine solche Reaktionsführung erlaubt die Anwendung einer höheren Reaktionstemporatur, wodurch die zunächst geringere Raumausbeute durch eine erhöhte Zeitausbeute wieder wettgemacht wird.

Die vorliegende Erfindung betrifft demnach ein Verfahren zur Herstellung von Cyclopropylamin durch Umsetzung von Cyclopropancarbonsäureamid mit Alkalihypohalogenit in Wasser als Reaktionsmedium (Hofmann-Abbau) und anschließende Behandlung des Umsatzungsgemisches mit Alkalihydroxid, das dadurch gekennzeichnet ist, daß man (a) das Cyclopropancarbonsäureamid in Form einer Lösung einsetzt, (b) das Umsetzungsgemisch zur Behandlung mit Alkalihydroxid in eine konzentrierte Alkalihydroxidlösung mit einem Überschuß an Alkalihydroxid von 1,1-4 Mol pro Mol Amid einträgt, wobei die Umsetzung bei 10-35°C erfolgt und alle Reaktionsschritte im homogenen Reaktionssystem durchgeführt werden, und (c) das Cyclopropylamin aus dem hierbei erhaltenen Reaktionsgemisch destillativ abtrennt.

Das erfindungsgemäße Verfahren wird bei 10-35°C, bevorzugt bei 10-25°C, besonders bevorzugt bei 15-20°C durchgeführt.

Die Umsetzung erfolgt durch Zusatz einer im allgemeinen stöchiometrischen Menge eines Alkalihypohalogenits. Als Alkalihypohalogenit sei beispielsweise Natriumhypochlorit, Natriumhypobromit, Kaliumhypochlorit und Kaliumhypobromit, bevorzugt Natriumhypochlorit, genannt.

Erfindungsgemäß ist es möglich, entgegen der Lehre des Standes der Technik das Umsetzungsgemisch zur Behandlung mit Alkalihydroxid in eine konzentrierte Alkalihydroxidlösung einzutragen, statt umgekehrt die Alkalihydroxidlösung zum Umsetzungsgemisch unter Kühlung zuzugeben. Hierbei braucht nach der erfindungsgemäßen Variante nicht gekühlt zu werden. Als Alkalihydroxid sei beispielsweise Natriumhydroxid oder Kaliumhydroxid, bevorzugt Natriumhydroxid, genannt. Diese Variante des Eintragens des Umsetzungsgemisches in eine konzentrierte Alkalihydroxidlösung bringt erhebliche Vorteile an Arbeitssicherheit, da die exotherme Hydrolyse und Abspaltung von $CO_2$ jederzeit durch die Geschwindigkeit des Eintragens kontrolliert werden kann. Der große Überschuß an Alkalihydroxid (1,1-4 Mol, bevorzugt 1,5-3 Mol pro Mol Amid) führt weiterhin zu einem vollständigen Ablauf der Reaktion, wobei unerwünschte Nebenreaktionen weitgehend unterdrückt werden können. Insbesondere wird eine unerwünschte Hydrolyse von Säureamid kaum oder gar nicht beobachtet.

Entgegen der Lehre der Technik hat sich also gezeigt, daß überraschenderweise das Arbeiten bei höheren Temperaturen nicht nur die Arbeitssicherheit erhöht, sondern auch Nebenreaktionen unterdrückt und damit Minderausbeuten vermeidet. Wichtig ist hierzu die erfindungsgemäße Beobachtung, daß im homogenen Reaktionssystem gearbeitet werden muß.

Nach dem Eintragen des Umsetzungsgemisches in die konzentrierte Alkalihydroxidlösung wird das Cyclopropylamin aus dem hierbei erhaltenen Gemisch destillativ abgetrennt. Entgegen der Lehre des Standes der Technik kann hierbei der größte Teil des Cyclopropylamins in einer weitgehend wasserfreien Form erhalten werden (0,1-5 Gew.-% Wasser).

Als der größte Teil des in nahezu wasserfreier Form erhältlichen Cyclopropylamins seien etwa 80-95 % des insgesamt erhaltenen Cyclopropylamins genannt. Der Rest fällt in Form eines Cyclopropylamin/Wasser-Destillats an, das in die Destillation zurückgeführt oder separat auf höherkonzentriertes Cyclopropylamin aufgearbeitet werden kann.

Die in homogener Phase durchführbare Umsetzung im erfindungsgemäßen Verfahren erlaubt auch die Durchführung in kontinuierlich arbeitenden Apparaturen; hierbei fallen stets nur geringe Mengen an vorübergehend instabilen Lösungen an, so daß die Arbeitssicherheit und die Ausbeute bei kontinuierlicher Arbeitsweise weiter gesteigert werden können.

Beispiel 1

299,6 g 99,3 %iges Cyclopropancarbonsäureamid wurden in 2100 g Wasser gelöst. Bei 10-20°C wurden innerhalb von 40 Minuten 1954 g 13,3 %ige NaOCl-Lösung zudosiert. Man ließ 1 Stunde bei 20°C nachrühren und überführte diese Lösung in eine Destillationsapparatur, in der sich bei 20°C 1344 g 50 %ige NaOH befanden. Während des Zupumpens stieg die Temperatur auf ca. 70°C an und wurde anschließend durch zusätzliches Heizen auf Siedetemperatur gebracht.

Ab 88°C Sumpftemperatur gewann man Cyclopropylamin vom Siedepunkt 49°C. Die Sumpftemperatur stieg kontinuierlich bis 108°C an, wobei nach der Gewinnung des Hauptanteils von Cyclopropylamin (ca. 80 % der theoretischen Ausbeute) die Kopftemperatur sehr schnell auf ca. 98°C stieg und noch eine wäßrige Cyclopropylaminlösung (ca. 15 % der theoretischen Ausbeute) anfiel, die beim nächsten Ansatz wieder aufdestilliert werden konnte.

Ausbeute:

```
I)  164,3 g mit 1,5 % Wasser

                    = 81,7 % der theor. Ausb.

II)  675 g mit 28,2 g Cyclopropylamin

                    = 14,1 % der theor. Ausb.
      _____

                    95,8 % der theor. Ausb.
```

Fraktion I erhielt man bei einer Kopftemperatur von 49-50°C, Fraktion II bei 50-100°C.

Beispiel 2

Eine Apparatur bestand aus 2 Rührkolben und einer separaten Destillationskolonne mit üblicher Ausrüstung. Kolben 1 wurde über 2 Pumpen mit einer Cyclopropancarbonsäureamid-Lösung und NaOCl-Lösung beschickt. Der Inhalt von Kolben 1 wurde über einen Überlauf in Kolben 2 gebracht; Kolben 2 wurde über die 3. Pumpe mit Natronlauge beschickt.

Nach Erreichen des stationären Zustandes (15 Minuten Vorlaufzeit) wurden folgende Mengen innerhalb von 30 Min. zur Reaktion gebracht:

171,7 g 99 %iges Cyclopropancarbonsäureamid wurden, in 888,2 g Wasser gelöst, gleichzeitig mit 1069,6 g 13,9 % NaOCl-Lösung bei etwa 18°C in den Kolben 1 eingepumpt; die Verweilzeit betrug 6 Minuten (Verweilzeit 1). Anschließend wurde mittels einer 3. Pumpe die daraus resultierende Mischung zusammen mit 711,0 g 45 %iger NaOH bei Temperaturen unterhalb 30°C und einer Verweilzeit von 2,5 Minuten im Kolben 2 vermischt (Verweilzeit 2) und in der Destillationsapparatur bis zum Beginn der diskontinuierlichen Destillation gesammelt.

Bei der Destillation erhielt man zuerst reines Cyclopropylamin (Sdp.: 49°C), dessen Wassergehalt durch das Rücklaufverhältnis bestimmt wurde (Rücklaufverhältnis 1:1 ergab ca. 2 - 3 % Wassergehalt, Rücklaufverhältnis 3:1 ergab ca. 1 % Wassergehalt), bevor im Bereich von 50-99°C eine wasserhaltige Fraktion anfiel, die bei 99°C nur noch ca. 1 - 2 % Cyclopropylamin enthielt.

Aus dieser Fraktion ließ sich Cyclopropylamin entweder durch nochmalige Feindestillation erhalten oder nach HCl-Zusatz als Hydrochlorid durch Abziehen des Wassers isolieren.

Ausbeute: 97,6 g Cyclopropylamin (CPA) = 1,71 Mol = 85,5 % der theoret. Ausbeute (isoliert als 100,1 g CPA mit 2,5 % Wassergehalt, Rücklaufverhältnis = 1:1, Siedepunkt 49°C) und 24,9 g CPA x HCl = 0,26 Mol = 13,0 % der theoret. Ausbeute (wasserhaltige Fraktion, 50-100°C). Summe = 98,5 % der theoret.

Ausbeute.

Beispiel 3

Die benutzte Apparatur bestand aus einem Reaktionsrohr (350 ml Volumen) mit Vormischeinrichtung und Kühlung und 2 Pumpen für die Zuführung von Cyclopropancarbonsäureamid-Lösung und von NaOCl-Lösung. Zugeordnet war eine Destillationsapparatur, die die NaOH als Vorlage enthielt (gekühlt auf unter 28°C), ausgerüstet mit einer 60 cm-Füllkörperkolonne, eingestellt auf ein Rücklaufverhältnis 1:1, 4 Ltr.-4-Halskolben, Heizbad, Innenthermometer, mech. Rührer, Einleitrohr.

171,7 g 99 %iges Cyclopropancarbonsäureamid wurden, in 888,2 g Wasser gelöst, gleichzeitig mit 1047,0 g 14,2 %iger NaOCl-Lösung bei einer Temperatur von 14°C gemischt und bei einer Verweilzeit von 4,25 Min. (VZ 1) zur Reaktion gebracht. Anschließend lief die Lösung in die gekühlte Vorlage von 711,0 g 45 %iger NaOH, wobei die Temperatur durch Kühlen unter 25°C gehalten wurde.

Die Destillation und Produktisolierung erfolgte wie im Beispiel 2.

Ausbeute: 100,6 g CPA = 1,77 Mol = 88,2 % der theoretischen Ausbeute (isoliert als 104,2 g CPA mit 3,5 % $H_2O$, Rücklaufverh. 1:1, Siedepunkt 49-50°C).

13,3 g CPA x HCl = 0,15 Mol = 7,1 % d.Th.

Summe = 95,5 % d.Th.

Beispiel 4 (zum Vergleich)

Die Reaktion führte man analog Beispiel 1 durch, allerdings wurde das Cyclopropancarbonsäureamid nicht in Wasser gelöst, sondern man arbeitete mit einer Suspension: statt 2100 g Wasser verwendete man 700 g Wasser. Die Aufarbeitung erfolgte wie in Beispiel 1.

Ausbeute:

```
I)  129,1 g mit 1,3 % Wasser = 61,1 % d.Th.
II) 652 g mit 23,4 g Cyclopropylamin =
                          11,7 % d.Th.
                          72,8 % d.Th.
```

**Patentansprüche**

1. Verfahren zur Herstellung von Cyclopropylamin durch Umsetzung von Cylcopropancarbonsäureamid mit Alkalihypohalogenit in Wasser als Reaktionsmedium (Hofmann-Abbau) und anschließende Behandlung des Umsetzungsgemisches mit Alkalihydroxid, dadurch gekennzeichnet, daß man (a) das Cyclopropancarbonsäureamid in Form einer Lösung einsetzt, (b) das Umsetzungsgemisch zur Behandlung mit Alkalihydroxid in eine konzentrierte Alkalihydroxidlösung mit einem Überschuß an Alkalihydroxid von 1,1-4 Mol pro Mol Amid einträgt, wobei die Umsetzung bei 10-35°C erfolgt und alle Reaktionsschritte im homogenen Reaktionssystem durchgeführt werden, und (c) das Cyclopropylamin aus dem hierbei erhaltenen Reaktionsgemisch destillativ abtrennt.

2. Verfahren nach Anspruch 1, dadurch gekennzeichnet, daß die Umsetzung bei 10-25°C, bevorzugt bei 15-20°C, durchgeführt wird.

3. Verfahren nach Anspruch 1, dadurch gekennzeichnet, daß als Alkalihydroxid Natriumhydroxid eingesetzt wird.

4. Verfahren nach Ansprüchen 1 bis 3, dadurch gekennzeichnet, daß die Umsetzung kontinuierlich durchgeführt wird.

## Claims

1. Process for the preparation of cyclopropylamine by reaction of cyclopropanecarboxamide with alkali metal hypohalite in water as a reaction medium (Hofmann degradation) and subsequent treatment of the reaction mixture with alkali metal hydroxide, characterized in that (a) the cyclopropanecarboxamide is employed in the form of a solution, (b) the reaction mixture for the treatment with alkali metal hydroxide is introduced into a concentrated alkali metal hydroxide solution with an excess of alkali metal hydroxide of 1.4-4 mol per mole of amide, the reaction being carried out at 10-35°C and all reaction steps being carried out in a homogeneous reaction system, and (c) the cyclopropylamine is separated off by distillation from the reaction mixture obtained in this case.

2. Process according to Claim 1, characterized in that the reaction is carried out at 10-25°C, preferably at 15-20°C.

3. Process according to Claim 1, characterized in that sodium hydroxide is employed as the alkali metal hydroxide.

4. Process according to Claims 1 to 3, characterized in that the reaction is carried out continuously.

## Revendications

1. Procédé de préparation de cyclopropylamine par réaction d'amide d'acide cyclopropane-carboxylique avec un hypohalogénite de métal alcalin dans l'eau comme milieu réactionnel (dégradation d'Hofmann) et par traitement subséquent du mélange réactionnel par un hydroxyde de métal alcalin, caractérisé en ce que (a) on utilise l'amide d'acide cyclopropane-carboxylique sous forme d'une solution, (b) on introduit le mélange réactionnel en vue du traitement par l'hydroxyde de métal alcalin dans une solution concentrée d'hydroxyde de métal alcalin contenant un excédent d'hydroxyde de métal alcalin de 1,1 à 4 moi par mole d'amide, étant entendu que la réaction a lieu entre 10 et 35°C et que toutes les étapes de la réaction sont mises en oeuvre dans le système réactionnel homogène et (c) on sépare par distillation la cyclopropylamine du mélange réactionnel ainsi obtenu.

2. Procédé selon la revendication 1, caractérisé en ce qu'on met en oeuvre la réaction entre 10 et 25°C, de préférence entre 15 et 20°C.

3. Procédé selon la revendication 1, caractérisé en ce qu'on utilise comme hydroxyde de métal alcalin l'hydroxyde de sodium.

4. Procédé selon les revendications 1 à 3, caractérisé en ce qu'on met en oeuvre la réaction en continu.